# EUROPEAN PATENT SPECIFICATION

(11) **EP 2 874 668 B1**
(45) Date of publication and mention of the grant of the patent: **19.04.2017**
(21) Application number: 13733016.3
(22) Date of filing: 04.07.2013
(51) Int. Cl.: A61L 2/18, B65B 55/10

(54) **STERILIZATION CHAMBER WITH INTERNAL FANS**
STERILISATIONSKAMMER MIT INTERNEN LÜFTERN
CHAMBRE DE STÉRILISATION AVEC VENTILATEURS INTERNES

(30) Priority: 17.07.2012 SE 1250870
(43) Date of publication of application: 27.05.2015
(73) Proprietor: Tetra Laval Holdings & Finance SA, 1009 Pully (CH)
(72) Inventor: ANDERSSON, Jan, S-271 94 Ystad (SE); SAEIDIHAGHI, Arash, 247 35 Lund (SE)
(74) Representative: Tetra Pak - Patent Attorneys SE
(86) International application number: PCT/EP2013/064176
(87) International publication number: WO 2014/012799

(56) References cited:
- EP-A1- 1 050 467
- WO-A1-2010/008334
- GB-A- 1 375 303

## Description

### Technical field of the invention

This invention relates to a sterilization chamber for sterilizing a web of packaging material passing through the chamber. The invention also relates to a method for sterilizing a web of packaging material, comprising the step of passing the web through a sterilization chamber, and subjecting the web to heat.

### Background art

Within packaging technology, use has long been made of packages for packing and transporting products such as milk, juice and other beverages. A large group of these packages is produced from a laminated packaging material comprising a core layer of, for example, paper or paperboard and an outer, liquid-tight coating of thermoplastic material on at least that side of the core layer which forms the inside of the package. Sometimes the material also includes a gas barrier, for example in the form of an aluminum layer.

WO 2010/008334 discloses a device for measurement of concentration of an oxidizable gas. The device comprises a catalyst which is surrounded by insulation inside a housing. The device further comprises at least two thermometers, one of which is located at the inlet of the device, and one being located inside the catalyst. The device also has means for calculating a concentration based on the temperature measurements. A sterilization chamber and a filling machine having a device according to the main claim are also disclosed.

GB 1375303 discloses an apparatus for controlling the atmosphere of a chamber in an aseptic packaging machine. In the machine a plastics coated paper web is withdrawn from a roll and passed through a bath filled with sterilizing liquid. Thereafter, the paper web is fed through seals and into a sterile chamber where it is sprayed with sterile hot air from a blowing device to free it from liquid residues. Finally, it is formed into a flexible tube by forming members.

EP 1050467 discloses a unit for sterilizing strip packaging material on a packaging machine for packaging pourable food products. The unit has a bath for containing a sterilizing agent in which the packaging material is fed continuously, an aseptic chamber having an input connected to an output of the bath, and an auxiliary recirculating circuit having a blower for aspirating air from the aseptic chamber. Two nozzles are located close to the input of the aseptic chamber to direct a jet of sterile air onto an intermediate longitudinal portion of the packaging material having preapplied opening devices.

Such packaging containers are often produced in that a web of packaging material is formed into a tube by overlappingly sealing the longitudinal edges of the web. The tube is continuously filled with a product and then transversally sealed and formed into cushions. The sealing is made along narrow transverse mutually spaced apart sealing zones. The transverse sealing of the tube takes place in a per se known manner substantially at right angles to the longitudinal direction of the tube. The sealed-off portions of the tube thus containing contents are thereafter separated from the tube by means of incisions in these sealing zones. Afterwards, the packages may be formed into for example parallelepiped packages by additional folding and sealing operations. This technology of forming a tube from a web is well known per se and will not be described in detail.

To extend the shelf-life of the products being packed it is prior known to sterilize the web before the forming and filling operations. Depending on how long shelf-life is desired and whether the distribution and storage is made in chilled or ambient temperature, different levels of sterilization can be chosen. One way of sterilizing a web is chemical sterilization using for example a bath of hydrogen peroxide.

After passing the sterilization bath, the packaging material web is normally subjected to heat in order to sterilize the web additionally and/or to remove excess sterilizing agent. In prior art, some removal of excess sterilizing agent is performed by leading the material web between a pair of squeegee rollers, and thereafter through a heating chamber, in which the web is heated by heating elements so that the agent is evaporated.

In some case, only heating elements are provided for evaporation of the sterilization agent, but this can be inconvenient. Firstly, it may be difficult to obtain a homogenous heating of the material web and secondly, in the case of an unexpected shutdown of the process, material web in the heating chamber may be subjected to too much heat, resulting in blistering of the thermoplastic material forming the liquid-tight coating of the container and other constructional damages.

### Summary of the invention

It is an object of the present invention to mitigate at least some of the above problems. This object is achieved with a sterilization chamber for sterilizing a web of packaging material passing through the chamber, comprising an internal fan with a heater for driving a circulating heated gas flow inside the chamber and towards and around the packaging material web.

The purpose is also achieved with a method for sterilizing a web of packaging material, comprising the step of passing the web through a sterilization bath and then a sterilization chamber, and driving a heated gas flow through the sterilization chamber with an internal fan with a heater at the outlet.

The characteristics given to the sterilization chamber above are preferably also comprised in the method of sterilizing a web.

### Brief Description of the Drawings

A sterilization chamber according to the present invention will be more fully understood from the following non-limiting example of a preferred embodiment, under reference to the accompanying drawings, in which:
Fig. 1 is a schematic sectional view of a sterilization chamber with a packaging material web threaded there through, subsequent to being brought through a peroxide bath, according to the invention,
Fig. 2 is a schematic front view of the sterilization chamber of Fig. 1, and
Fig. 3 is a schematic perspective view of sidewalls that are mounted in a sterilization chamber of the invention.

### Detailed Description of a Preferred Embodiment

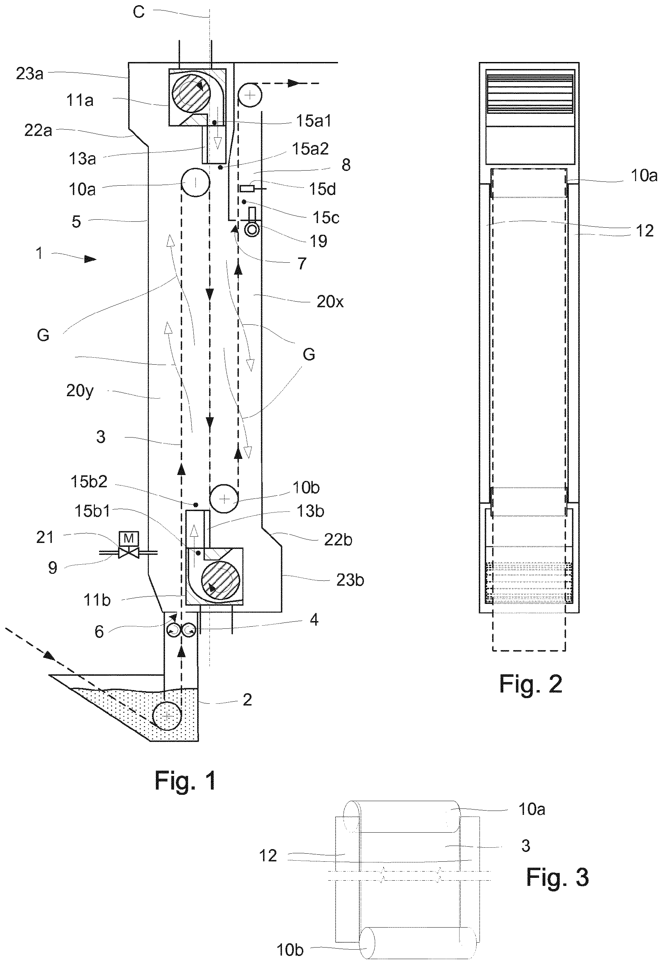

In a production line where containers suitable for beverage are produced, a sterilization chamber 1 is preceded by a sterilization bath 2 containing a sterilizing agent, such as hydrogen peroxide, as can be seen in Fig. 1. After passing through the sterilization bath 2, the web 3 of packaging material, still carrying a film of sterilizing agent, is led through the sterilization chamber 1 where the remaining sterilizing agent will be evaporated in order to create a vapor concentration of sterilizing agent inside the sterilization chamber. Prior to entering the sterilization chamber 1, the web 3 passes a pair of squeegee rollers 4, where sterilizing agent is evenly spread out over the entire width of the web 3 and excess sterilizing agent is removed.

The sterilization chamber 1 is formed by an outer body 5, constructed from stainless steel. The body 5 is provided with a body inlet 6 and a body outlet 7, both in the shape of slots, where the packaging material web 3 enters and leaves, respectively, the sterilization chamber 1. The body outlet 7 leads out into a sterilization chamber outlet 8.

Two rollers 10a, 10b are provided for guiding the packaging material web 3 inside the sterilization chamber, upper roller 10a at the upper part of the sterilization chamber 1 and lower roller 10b at the lower part of the sterilization chamber 1. In this way, the residence time is increased for the web 3 inside the sterilization chamber 1.

Two tangential fans 11a, 11b, or cross-flow fans, are further arranged at the top and bottom of the sterilization chamber 1. The outlet of the lower fan 11b is directed towards the upper roller 10a, and the outlet of the upper fan 11a is directed towards the lower roller 10b. The inlets to the fans 11a, 11b are located at the side of the sterilization chamber, facing in opposite directions.

Sidewalls or baffles 12 are arranged on the left and right walls of the sterilization chamber 1, see Fig. 2, and they project outwards towards the upper and lower rollers 10a, 10b. These sidewalls 12 generally start from the right side of the upper roller 10a, as seen in Fig. 1, and end at the left side of the lower roller 10b. During operation, with the packaging material web 3 threaded through the sterilization chamber 1, the sidewalls 12 divide the sterilization chamber 1 into two regions 20x, 20y. In Fig. 3, a schematic view of the baffles 12 can be seen perspective.

Upper 13a and lower 13b electrical heaters are arranged at the outlet of the upper 11a and lower 11b tangential fans, for heating the gas flowing there through. The heaters 13a, 13b are configured to have a small mass, in order to have small thermal inertia. The heaters generally comprise heating strips formed from high-resistance electrical wire or strip, in a per se known way. The exact design of the heater is not part of the invention.

A number of thermocouples 15a1, 15a2, 15b1, 15b2, 15c are arranged inside the sterilization chamber 1, in order to allow monitoring of different parameters, such as e.g. the heating efficiency of the heaters 13a, 13b. Two thermocouples 15a1, 15b1 are arranged at the inlets of the heaters 13a, 13b, two thermocouples 15a2, 15b2, are arranged at the outlets of the heaters 13a, 13b, and one thermocouple 15c is arranged at the outlet of the sterilization chamber 1.

A further thermocouple 15d is arranged in the sterilization chamber outlet 8, for measuring the surface temperature of the packaging material web 3. This is done by mounting the thermocouple 15d in sliding contact with the web 3. The thermocouple 15d is connected to a control system (not shown), for controlling the operation of the sterilization chamber 1.

A concentration sensor 19 is connected between the sterilization chamber 1 and the outlet chamber 8, and a small flow of gas is directed there through, in order to obtain a measurement of the concentration of sterilizing agent in the gas in the sterilization chamber 1. Sterilizing agent is brought to the sterilization chamber 1 by the packaging material web 3 passing through the sterilization bath 2, and sterile air is supplied through a separate sterile air inlet 9. This is only shown schematically in Fig. 1, and it can be located in different places. The air supply through the air inlet 9 is controlled by a motorized valve 21, governed by the control system. The control system also receives a signal from the concentration sensor 19.

The body 5 of the sterilization chamber 1 is provided with angled walls 22a, 22b at the top and bottom of the lateral walls facing the fan inlets. These angled walls 22a, 22b move out a top 23a and bottom 23b lateral wall to a certain distance from the fan inlets, creating a larger top and bottom volume at the fan inlets. In one embodiment, the top 23a and bottom 23b lateral walls are located at a distance from impellers of the fans 11a, 11b being equal to or greater than the diameter of said impellers.

During operation of the sterilization chamber 1, the packaging material web 3 is pulled by a downstream jaw system, having sealing jaws for forming, sealing and cutting off individual pillow packages (not shown). The web is first brought through the sterilization bath 2, which is partly filled with sterilizing agent, such that the entire width of the web 3 is coated with sterilizing agent on both main surfaces. Any excess of sterilizing agent is removed by the squeegee rollers 4, just upstream of the sterilization chamber inlet 6, and the packaging material web 3 then enters the sterilization chamber 1. The web from there runs to the upper roller 10a and is then turned 180 degrees to be led down to the lower roller 10b, before being turned again towards the sterilization chamber outlet 7.

The material web 3 hence passes a certain length of the heating chamber 1 three times, guided by the rollers 10a, 10b. The central passage of the web 3 inside the sterilization chamber runs generally in alignment with the sidewalls or baffles 12, along a centerline C.

The fans 11a, 11b are at the same time operated to drive a gas flow towards a respective roller 10b, 10a. The upper fan 11a drives a gas flow through an upper heater 13a, and then down into a back region 20x between two sections of running web, to the right of the centerline C, as seen in Fig. 1. The gas flow is restricted downward by the lower roller 10b and by the web 3, and this forces the flow towards the right and left walls of the sterilization chamber 1 (as seen in Fig. 1). The sidewalls or baffles 12 present a hindrance for the flow to enter a front region 20y, and the flow is instead lead towards the back wall, to the right in Fig.1, and finally downward towards the inlet of the bottom fan 11b, as is shown by the lines G with hollow arrow heads.

A corresponding flow occurs for the lower fan 11b, but with the flow instead being diverted towards the other outer side of the sterilization chamber 1, the front region 20y to the left as seen in Fig. 1, and towards the inlet of the upper fan 11a.

In this way, the gas inside the sterilization chamber is kept in continuous driven motion, and the gas temperature is controlled by the upper 13a and lower 13b heaters, through the use of thermocouples 15a1, 15a2, 15b1, 15b2, 15c and a control system. By controlling the gas temperature inside the sterilization chamber, and by driving the convection with the fans, the vaporization of sterilizing agent from the packaging material web can be controlled. The convection assists in transferring heat to the packaging material web 3 and the sterilizing agent thereupon. It is hence possible to lower the maximum temperature in the sterilization chamber 1 and still maintain an effective vaporization of sterilizing agent.

An important parameter is the surface temperature of the packaging material web 3. This is measured by the further thermocouple 15d, and this value is in one embodiment used for controlling the power to the heaters 13a, 13b. The temperature of the web 3 should then be high enough to achieve effective sterilization and evaporation of sterilizing agent, and low enough to prevent blistering of the packaging material web 3. In one embodiment, the signal from the further thermocouple 15d is used for controlling the power to the heaters 13a, 13b, and for hence controlling the gas temperature inside the sterilization chamber 1. The gas temperature inside the chamber 1 is controlled by the thermocouples 15a1, 15a2, 15b1, 15b2 to be within acceptable limits, for safe and reliable operation.

The concentration of vaporized sterilizing agent may be important for obtaining an effective killing of potential harmful bacteria or microbes. This concentration is controlled by measuring the concentration of sterilizing agent inside the sterilization chamber, and supplying more air through the air supply 9 as needed. It is hence possible to control the gas concentration separately from the convection, inside the sterilization chamber 1.

During normal operation of the sterilization chamber 1, the temperature at the inlet to the fan(s) is between 90 and 120 °C, and at the fan outlet between 120 and 150 °C. With a packaging material web speed of about 0.5 m/s, this leads to a web temperature of between 65 and 85 °C, depending on the average temperature inside the sterilization chamber. The airflow into the chamber 1 is about 15 to 20 kg/hour (corresponding to the outflow from the chamber), and the re-circulated gas flow inside the chamber corresponds to about 800 to 1200 kg/hour. The hydrogen peroxide vapor concentration inside the chamber 1 is about 10000 to 20000 ppm. This is achieved when the hydrogen peroxide application on the web 3 is about 1.3 g/m2. The hydrogen peroxide concentration in the sterilization bath 2 is about 35%.

It is possible to arrange only one fan 11a inside the sterilization chamber 1, and to arrange baffles or walls such that the flow from the outlet of the fan 11a is directed towards and around the packaging material web 3 and finally towards the inlet of said fan 11a. In this way, the convection can be driven in a controlled way. It is also possible to have more than two fans, all being arranged to blow a flow from an outlet into an inlet of the downstream fan, in order to create a strong, coupled flow, at least some of the fans comprising a heater in the outlet thereof.

The further thermocouple 15d, being in sliding contact with the packaging material web 3, may be replaced by a non-contact thermometer, such as an infrared thermometer or similar.

The air supply valve 21 can be any valve that can be used for controlling the air flow, and can also be a manually activated valve. The location of the valve 21 is not critical, so it can be placed at several locations in the body 5.

As sterilizing agent, hydrogen peroxide is most commonly used but other sterilizing agents are also possible.

Tangential fans or cross-flow fans are disclosed in the embodiments of this invention, but it is possible to use also other types of fans, such as axial flow fans or centrifugal fans.

The front wall of the sterilization chamber 1 is the left wall as seen in Fig. 1, which means that the back wall is the right wall in this figure. The left and right walls are hence to the left and right in Fig. 2, which is shown with the front wall facing the viewer.

## Claims

1. A sterilization chamber (1) for sterilizing a web (3) of packaging material passing through the sterilization chamber (1), comprising an air-distribution system and heaters (13a, 13b) arranged to heat the web (3), **characterized in that** two fans (11a, 11b) are provided at either end of the sterilization chamber (1), for driving a circulating flow therein, such that the flow from the outlets of each fan (11a, 11b) is lead towards the inlet of the respective other fan (11b, 11a), and where one heater (13a, 13b) is arranged at the outlet of each fan (11a, 11b).

2. A sterilization chamber (1) according to claim 1, wherein sidewalls (12) are arranged inside the sterilization chamber, for guiding the flow from the outlet of one fan (11a) towards the inlet of the other fan (11b), and vice versa.

3. A sterilization chamber (1) according to claim 2, wherein the sidewalls (12), during operation, cooperate with the web (3) for sealing off a centerline (C) such that the flow from the outlet of one fan (11a) is directed towards and around the packaging material web (3), and subsequently towards the inlet of the other fan (11b), and vice versa.

4. A sterilization chamber (1) according to claim 1, wherein a first thermocouple (15a1, 15b1) is arranged at an inlet of said heaters (13a, 13b), and a second thermocouple (15a2, 15b2) is arranged at the outlet of said heaters (13a, 13b), respectively.

5. A sterilization chamber (1) according to claim 4, wherein a further thermocouple (15d), measuring the surface temperature of the packaging material web (3), is connected to a control system, for controlling the power supplied to the heaters (13a, 13b).

6. A sterilization chamber (1) according to claim 1, wherein each fan (11a, 11b) is a tangential fan or cross-flow fan.

7. A sterilization chamber (1) according to claim 1, wherein each heater (13a, 13b) is an electrical heater.

8. A sterilization chamber (1) according to claim 1, wherein baffles (12) are arranged therein such that the flow from the outlet of the fan (11a; 11b) is directed to the inlet of said fan (11a; 11b).

9. A sterilization chamber (1) according to claim 1, wherein a separate air supply (9) is arranged in a body (5) of the sterilization chamber (1).

10. sterilization chamber (1) according to claim 9, wherein the air supply (9) is controlled by a control system, connected to a motorized control valve (21).

11. A method of sterilizing a packaging material web (3), comprising the steps of
a) feeding a packaging material web (3) through a sterilizing agent bath, and then into a sterilization chamber (1),
b) driving a gas flow inside said chamber (1) with a fan (11a; 11b),
c) heating the air leaving the outlet of the fan (11a; 11b) with heaters (13a; 13b),
d) directing the flow from the outlet of the fan (11a; 11b) towards the packaging material web (3) and on towards the inlet of the fan (11a; 11b),
wherein the flow from the outlet of one fan (11a; 11b) is directed towards the inlet of another fan (11b, 11a) .

12. A method according to claim 11, wherein a power to the heater (13a; 13b) is controlled by a control system using the input from a thermometer (15d) measuring the surface temperature of the packaging material web (3).

13. A method according to claim 11, wherein a concentration of sterilizing agent is measured with a concentration sensor (19), arranged inside the sterilization chamber (1), and the concentration is controlled by regulating a valve (21) connected to a sterile air supply (9).

## Patentansprüche

1. Sterilisationskammer (1) zum Sterilisieren einer durch die Sterilisationskammer (1) verlaufenden Bahn (3) aus Verpackungsmaterial, umfassend ein Luftverteilungssystem und Heizungen (13a, 13b), angeordnet zum Heizen der Bahn (3), **dadurch gekennzeichnet, dass** zwei Lüfter (11a, 11b) an beiden Enden der Sterilisationskammer (1) vorgesehen sind zum Antreiben eines zirkulierenden Flusses darin, so dass der Fluss an den Auslässen jedes Lüfters (11a, 11b) zum Einlass des jeweiligen anderen Lüfters (11b, 11a) geführt wird, und wobei eine Heizung (13a, 13b) am Auslass jedes Lüfters (11a, 11b) angeordnet ist.

2. Sterilisationskammer (1) nach Anspruch 1, wobei Seitenwände (12) innerhalb der Sterilisationskammer angeordnet sind zum Führen des Flusses zum Auslass eines Lüfters (11a) zum Einlass des anderen Lüfters (11b) und umgekehrt.

3. Sterilisationskammer (1) nach Anspruch 2, wobei die Seitenwände (12) während des Betriebs mit der Bahn (3) zusammenarbeiten zum Abdichten einer Mittellinie (C), so dass der Fluss von dem Auslass eines Lüfters (11a) zu der Verpackungsmaterialbahn (3) und dort herum und danach zum Einlass des anderen Lüfters (11b) und umgekehrt geführt wird.

4. Sterilisationskammer (1) nach Anspruch 1, wobei ein erstes Thermoelement (15a1, 15b1) am Einlass der Heizungen (13a, 13b) angeordnet ist und ein zweites Thermoelement (15a2, 15b2) jeweils am Auslass der Heizungen (13a, 13b) angeordnet ist.

5. Sterilisationskammer (1) nach Anspruch 4, wobei ein weiteres Thermoelement (15d), das die Oberflächentemperatur der Verpackungsmaterialbahn (3) misst, an ein Steuersystem angeschlossen ist zum Steuern der an die Heizungen (13a, 13b) gelieferten Leistung.

6. Sterilisationskammer (1) nach Anspruch 1, wobei jeder Lüfter (11a, 11b) ein Tangentiallüfter oder ein Kreuzstromlüfter ist.

7. Sterilisationskammer (1) nach Anspruch 1, wobei jede Heizung (13a, 13b) eine elektrische Heizung ist.

8. Sterilisationskammer (1) nach Anspruch 1, wobei Leitbleche (12) darin angeordnet sind, so dass der Strom von dem Auslass des Lüfters (11a; 11b) zum Einlass des Lüfters (11a; 11b) gelenkt wird.

9. Sterilisationskammer (1) nach Anspruch 1, wobei eine separate Luftzufuhr (9) in einem Körper (5) der Sterilisationskammer (1) angeordnet ist.

10. Sterilisationskammer (1) nach Anspruch 9, wobei die Luftzufuhr (9) durch ein Steuersystem gesteuert wird, das an ein motorisiertes Steuerventil (21) angeschlossen ist.

11. Verfahren zum Sterilisieren einer Verpackungsmaterialbahn (3) umfassend die folgenden Schritte:
a) Zuführen einer Verpackungsmaterialbahn (3) durch ein Sterilisationsmittelbad und dann in eine Sterilisationskammer (1),
b) Antreiben eines Gasflusses innerhalb der Kammer (1) mit einem Lüfter (11a; 11b),
c) Heizen der den Auslass des Lüfters (11a; 11b) verlassenden Luft mit Heizungen (13a; 13b),
d) Lenken des Flusses von dem Auslass des Lüfters (11a; 11b) zur Verpackungsmaterialbahn (3) und weiter zum Einlass des Lüfters (11a; 11b),
wobei der Fluss von dem Auslass eines Lüfters (11a; 11b) zum Einlass eines weiteren Lüfters (11b, 11a) gelenkt wird.

12. Verfahren nach Anspruch 11, wobei eine Leistung zu der Heizung (13a; 13b) durch ein Steuersystem unter Verwendung der Eingabe von einem Thermometer (15d) gesteuert wird, das die Oberflächentemperatur der Verpackungsmaterialbahn (3) misst.

13. Verfahren nach Anspruch 11, wobei eine Konzentration eines Sterilisierungsmittels mit einem Konzentrationssensor (19) gemessen wird, in der Sterilisationskammer (1) angeordnet, und die Konzentration wird durch Regeln eines Ventils (21) gesteuert, das an eine sterile Luftversorgung (9) angeschlossen ist.

## Revendications

1. Chambre de stérilisation (1) permettant de stériliser une bande (3) d'un matériau d'emballage traversant la chambre (1), comprenant un système de distribution d'air et des éléments chauffants (13a, 13b) disposés pour chauffer la bande (3), **caractérisé en ce que** deux ventilateurs (11a, 11b) sont disposés à chaque extrémité de la chambre de stérilisation (1) pour y entraîner un flux en circulation, de sorte que le flux en sortie de chaque ventilateur (11a, 11b) soit conduit vers l'entrée de l'autre ventilateur respectif (11b, 11a), et un élément chauffant (13a, 13b) étant disposé au niveau de la sortie de chaque ventilateur (11a, 11b).

2. Chambre de stérilisation (1) selon la revendication 1, dans laquelle des parois latérales (12) sont disposées à l'intérieur de la chambre de stérilisation pour guider le flux de la sortie d'un ventilateur (11a) vers l'entrée de l'autre ventilateur (11b), et inversement.

3. Chambre de stérilisation (1) selon la revendication 2, dans laquelle les parois latérales (12), lors de l'utilisation, coopèrent avec la bande (3) pour étanchéifier une ligne médiane (C) de sorte que le flux en sortie d'un ventilateur (11a) soit dirigé vers la bande de matériau d'emballage (3) et autour d'elle, puis vers l'entrée de l'autre ventilateur (11b), et inversement.

4. Chambre de stérilisation (1) selon la revendication 1, dans laquelle, respectivement, un premier thermocouple (15a1, 15b1) est disposé au niveau d'une entrée desdits éléments chauffants (13a, 13b), et un second thermocouple (15a2, 15b2) est disposé au niveau de la sortie desdits éléments chauffants (13a, 13b).

5. Chambre de stérilisation (1) selon la revendication 4, dans laquelle un autre thermocouple (15d), mesurant la température de surface de la bande de matériau d'emballage (3), est connecté à un système de contrôle afin de contrôler l'alimentation des éléments chauffants (13a, 13b).

6. Chambre de stérilisation (1) selon la revendication 1, dans laquelle chaque ventilateur (11a, 11b) est un ventilateur tangentiel ou à écoulement perpendiculaire.

7. Chambre de stérilisation (1) selon la revendication 1, dans laquelle chaque élément chauffant (13a, 13b) est un élément chauffant électrique.

8. Chambre de stérilisation (1) selon la revendication 1, dans laquelle des déflecteurs (12) y sont disposés afin que le flux en sortie du ventilateur (11a, 11b) soit dirigé vers l'entrée dudit ventilateur (11a, 11b).

9. Chambre de stérilisation (1) selon la revendication 1, dans laquelle une alimentation d'air séparée (9) est disposée dans un corps (5) de la chambre de stérilisation (1).

10. Chambre de stérilisation (1) selon la revendication 9, dans laquelle l'alimentation d'air (9) est contrôlée par un système de contrôle connecté à une vanne de contrôle motorisée (21).

11. Procédé de stérilisation d'une bande de matériau d'emballage (3), comprenant les étapes consistant à :
a) faire passer une bande de matériau d'emballage (3) dans un bain d'agent stérilisant, puis dans une chambre de stérilisation (1) ;
b) entraîner un flux gazeux à l'intérieur de ladite chambre (1) au moyen d'un ventilateur (11a, 11b) ;
c) chauffer l'air en sortie du ventilateur (11a, 11b) au moyen d'éléments chauffants (13a, 13b) ;
d) diriger le flux de la sortie du ventilateur (11a, 11b) vers la bande de matériau d'emballage (3) et vers l'entrée du ventilateur (11a, 11b),
le flux en sortie d'un ventilateur (11a, 11b) étant dirigé vers l'entrée d'un autre ventilateur (11b, 11a).

12. Procédé selon la revendication 11, dans lequel une puissance fournie à l'élément chauffant (13a, 13b) est contrôlée par un système de contrôle au moyen de l'entrée provenant d'un thermomètre (15d) mesurant la température de surface de la bande de matériau d'emballage (3).

13. Procédé selon la revendication 11, dans lequel une concentration d'agent stérilisant est mesurée au moyen d'un capteur de concentration (19) disposé à l'intérieur de la chambre de stérilisation (1), et dans lequel la concentration est contrôlée en régulant une vanne (21) connectée à une alimentation d'air stérile (9).
